Europäisches Patentamt

(19) European Patent Office

Office européen des brevets

(11) Numéro de publication: **0 147 244**

**A1**

(12) # DEMANDE DE BREVET EUROPEEN

(21) Numéro de dépôt: **84400661.9**

(22) Date de dépôt: **04.04.84**

(51) Int. Cl.⁴: **A 61 K 9/54**
**A 61 K 31/40**

(30) Priorité: **23.12.83 FR 8320635**

(43) Date de publication de la demande:
**03.07.85 Bulletin 85/27**

(84) Etats contractants désignés:
**AT BE CH DE FR GB IT LI LU NL SE**

(71) Demandeur: **SOCIETE D'ETUDES SCIENTIFIQUES ET INDUSTRIELLES DE L'ILE DE FRANCE**
**46, Boulevard de Latour-Maubourg**
**F-75340 Paris Cedex 07(FR)**

(72) Inventeur: **Pitel, Guy**
**22, Parc de Midori**
**F-78350 Les LOges en Josas(FR)**

(74) Mandataire: **Gallochat, Alain**
**SOCIETE D'ETUDES SCIENTIFIQUES ET INDUSTRIELLES DE L'ILE DE FRANCE 46, Boulevard de Latour Maubourg**
**F-75340 Paris Cedex 07(FR)**

(54) **Nouvelles formes galéniques du sulpiride utilisables par voie orale.**

(57) Formes galéniques du Sulpiride comprenant des microgranules constitués par un grain neutre sur lequel est appliqué le sulpiride, puis une couche extérieure de polymères microporeux, en particulier un mélange de méthacrylates du type Eudragit (R).

La libération in vitro dans des milieux gastrique et intestinal artificiel montre une cinétique particulière de ces microgranules.

EP 0 147 244 A1

Croydon Printing Company Ltd

0147244

L'invention concerne des nouvelles formes galéniques du Sulpiride, utilisables par voie orale, ainsi que leur procédé de préparation.

Le Sulpiride, de formule développée N- (éthyl-1 pyrrolidinyl-2) méthyl méthoxy-2 sulfamoyl-5 benzamide, est connu comme étant un excellent neuroleptique désinhibiteur, ayant comme indication thérapeutique principale le traitement des psychoses aigües et chroniques, et ses caractéristiques et ses propriétés sont notamment décrites dans les brevets français n° 1.472.025, 4879 M et 5916 M.

Les médicaments actifs par voie orale contenant du Sulpiride se présentent actuellement sous la forme de comprimés, de gélules ou de solutés buvables mais, et les résultats exposés ci-après le démontrent, ces formes actuelles nécessitent une pluralité de doses quotidiennes et ont parfois un pourcentage réduit d'absorption par l'organisme.

On a par conséquent cherché à modifier la forme galénique, de façon à améliorer de façon sensible son acceptation par les patients, en réduisant le nombre de prises journalières.

La demi-vie plasmatique du principe actif Sulpiride étant d'environ 8 h 30, il apparait a priori possible de réaliser une forme galénique du produit, permettant de réduire la prise journalière moyenne à 2 gélules ou comprimés par jour, dosés en Sulpiride à 100 mg par gélules ou comprimés (avec une prise le matin et une prise le soir), sinon à 1 gélule ou 1 comprimé par jour, dosé à 200 mg de Sulpiride.

Pour cela, on a réalisé des microgranules à effet prolongé, selon les techniques classiques décrites notamment dans les brevets français n° 2.313.915 (Corneille) ou n° 2.453.642 (Labaz). Classiquement, on a utilisé des grains neutres dont la taille était d'environ 0,50 mm de diamètre, et constitués de 25 % d'amidon et de 75 % de saccharose, sur lesquels on a projeté en turbine le Sulpiride au moyen d'une solution alcoolique de polyvinylpyrrolidone, puis on a appliqué des fibres d'enrobage classiques, à base de polymère du type gomme laque, méthacrylates ou éthylcellulose, de façon à obtenir une cinétique de libération du principe actif Sulpiride in vitro dans des milieux gastriques et intestinaux pré-établie standard, conformément aux spécifications ci-après :

- 1ère heure : libération inférieure à 40 %
- 4ème heure : libération inférieure à 75 %
- 8ème heure : libération supérieure à 75 %

La libération à la première heure est effectuée dans un suc gastrique artificiel à pH 1,3, à la quatrième heure et à la huitième heure respectivement dans des sucs intestinaux artificiels à pH 4,5 et pH 7. Les conditions opératoires pour étudier la libération du principe actif sont connues et décrites dans les brevets précités.

On a ensuite réalisé avec ces microgranules des gélules et des comprimés dosés à 100 mg et on a étudié l'absorption du Sulpiride en déterminant les taux plasmatiques circulant du Sulpiride par radio-immunologie, puis on a comparé les résultats obtenus avec ceux issus de la forme gélules actuellement sur le marché.

Les résultats sont consignés dans le tableau A ci-après et montrent qu'avec les deux formulations de microgranules utilisés (forme I et forme II), la perte en taux de principe actif circulant est trop importante par rapport à celui de la forme normale en gélules (forme IV), pour permettre de réaliser un médicament à effet prolongé efficace.

A titre indicatif, pour l'homme de l'art, les formes I et II utilisées possèdaient les cinétiques suivantes de libération in vitro en milieux artificiels :

|  | forme I | forme II |
|---|---|---|
| Libération à la première heure dans un milieu de pH : 1,3 | 20,0 % | 29,2 % |
| Libération à la quatrième heure dans un milieu de pH : 4,5 | 68,6 % | 58,4 % |
| Libération à la huitième heure dans un milieu de pH : 7 | 90,8 % | 98,2 % |

Après l'étude d'une série de formulations à effet prolongé qui ont toutes donné des résultats incompatibles avec l'obtention d'une bonne forme orale bien absorbée, on a eu l'idée de conserver la forme microgranules (grains sensiblement sphériques de diamètre compris entre 0,2 et 2 mm) qui doit permettre en pratique une meilleure absorption du principe actif qu'une poudre classique de la gélule ou du comprimé, mais en ayant une cinétique de libération in vitro beaucoup plus proche de ces formes immédiates, c'est-à-dire libérant sensiblement 90 % du principe actif au bout d'une heure dans un milieu gastrique artificiel de pH 1,3.

0147244

De façon surprenante, les résultats des microgranules selon cette cinétique in vitro ont montré que, en fonction des taux plasmatiques comparatifs, cette forme III constituée de microgranules mis en gélules dosées à 100 mg permet d'obtenir une absorption double de celle obtenue avec les gélules classiques de Sulpiride (forme IV) tout en conservant une durée d'action légèrement supérieure et en réduisant considérablement d'un sujet à l'autre les écarts d'absorption. Cette amélioration de l'absorption du Sulpiride tant au niveau quantitatif qu'au niveau de sa régularité autorise une réduction des doses utilisées et du nombre des prises quotidiennes.

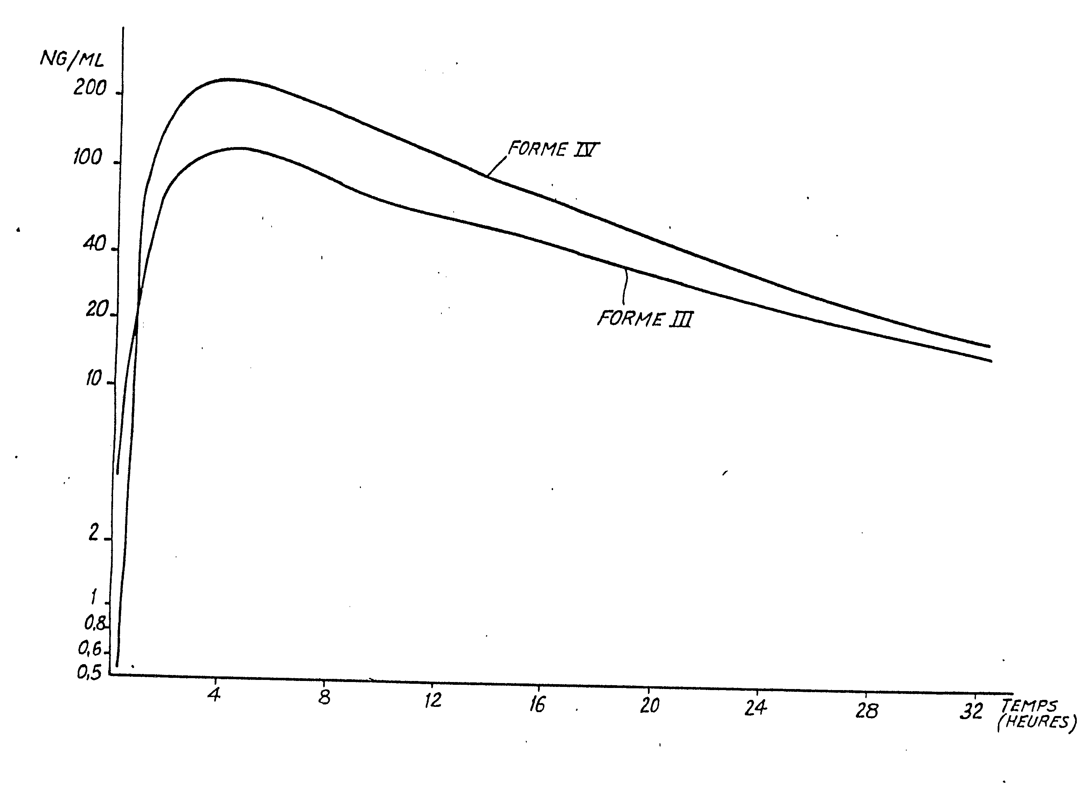

Ces résultats sont exprimés dans le Tableau A, ainsi que par la figure 1, montrant l'évolution des taux plasmatiques circulant exprimés en ng/ml de Sulpiride, entre 0 et 32 heures par comparaison entre la forme III et la forme IV.

Des expérimentations complémentaires au cours desquelles on a fait varier les enrobages externes des microgranules selon l'invention ont permis de déterminer que la forme préférentielle à absorption améliorée du Sulpiride devait répondre sensiblement aux conditions analytiques suivantes de libération in vitro :

. 15 minutes : libération comprise entre 35 et 50 % dans un milieu gastrique artificiel de pH 1,3

. 30 minutes : libération comprise entre 60 et 75 % dans un milieu gastrique artificiel de pH 1,3

. 60 minutes : libération comprise entre 80 et 95 % dans un milieu intestinal artificiel de pH 4,5

On note par ailleurs une libération globale supérieure à 90 % dans un milieu intestinal artificiel de pH 6,9

Comme exemple de réalisation, on a indiqué ci-après la formule de fabrication pour 2.500 gélules dosées à 100 mg de la forme III, ainsi que son procédé de fabrication.

On utilise 50 g de grains neutres constitués d'environ 25 % d'amidon et de 75 % de saccharose, de taille sensiblement égale à 0,50 mm de diamètre, sur lesquels on applique en turbine 250 g de Sulpiride incorporé dans une solution alcoolique (éthanol) de polyvinylpyrrolidone à 20 %.

Après séchage et tamisage, on a appliqué des couches extérieures composées de polymères méthacryliques commercialisés sous le nom d'Eudragit (R) par la société Röhm et Haas en solutions alcooliques à raison de 4 parties en poids d'Eudragit type L, pour 1 partie en poids d'Eudragit type RL.

On sèche ensuite avec du talc en faibles proportions, jusqu'à l'obtention de microgranules libérant sensiblement 100 % en une heure selon les conditions analytiques définies ci-dessus.

On sèche ensuite à l'étuve à 37°C pendant environ deux jours, puis on termine la couche externe par projection de 40g d'une solution éthanolique à 13,5 % d'Eudragit type RL. Le titre constaté est d'environ 730 mg de principe actif par gramme de microgranules.

Les microgranules ainsi obtenus répondent aux normes de libération in vitro définies ci-dessus, comme étant représentatives des formes galéniques nouvelles du Sulpiride selon l'invention.

Leurs stabilités accélérée et naturelle se sont révélées excellentes sans apparition de toxicité potentialisée.

Ils peuvent être utilisés en gélules, en comprimés ou en suspension, dans des milieux neutres.

Bien entendu, l'homme de l'art pourra introduire des variantes, notamment dans les proportions respectives des excipients et du principe actif, la composition des grains neutres et dans la nature des polymères constituant les couches externes, sans pour cela sortir du cadre de l'invention.

| Paramètres cinétiques | Concentration maximale moyenne (Cmax) en ng/ml | Temps correspondant au Cmax moyen (Tmax) moyen en heure | Aire sous la courbe observée (AUC) | |
|---|---|---|---|---|
| | | | 0-8 heures en ng/ml $^{-1}$ h | 0-24 heures en ng/ml $^{-1}$ h |
| Forme I | $75 \pm 40$ | $4 \pm 1,50$ | $3,40 \pm 75$ | $720 \pm 190$ |
| Forme II | $80 \pm 45$ | $3,90 \pm 1,40$ | $3,65 \pm 80$ | $780 \pm 200$ |
| Forme III | $315 \pm 65$ | $3,30 \pm 1,30$ | $1280 \pm 230$ | $2970 \pm 400$ |
| Forme IV | $150 \pm 60$ | $3,20 \pm 1,30$ | $715 \pm 320$ | $1565 \pm 430$ |

REVENDICATIONS

1. Nouvelles formes galéniques du Sulpiride, utilisables par voie orale, comprenant des microgranules constitués par un grain neutre du type mélange de saccharose, d'amidon, de lactose, de talc ou d'acide stéarique, sur lequel est appliqué le Sulpiride, puis une couche extérieure de polymères microporeux du type éthylcellulose, gomme laque, méthacrylates, acétophtalate de cellulose ou polyvinylpyrrolidone, caractérisées en ce que la libération in vitro du Sulpiride dans des milieux gastrique et intestinal artificiel :

. Au bout de 15 minutes est sensiblement comprise entre 35 et 50 %, dans un milieu de pH 1,3

. Au bout de 30 minutes est sensiblement comprise entre 60 et 75 % dans un milieu de pH 1,3

. Au bout de 60 minutes est sensiblement comprise entre 80 et 95 % dans un milieu de pH 4,5

. A plus de 2 heures est sensiblement supérieure à 90 %, dans un milieu de pH 6,9

2. Nouvelles formes galéniques du Sulpiride, selon la revendication 1, caractérisées en ce que la couche externe desdits microgranules est composée d'un ou plusieurs méthacrylate du type Eudragit (R).

3. Nouvelles formes galéniques du Sulpiride, selon la revendication 1, caractérisées en ce que la couche externe desdits microgranules est composée de mélanges de méthacrylates comportant environ 3 à 4 parties en poids de méthacrylates du type Eudragit (R)L et 0,5 à 2 parties en poids de méthacrylates du type Eudragit (R)RL.

4. Nouvelles formes galéniques du Sulpiride, selon une des revendications 1 à 3, caractérisées en ce que la couche contenant le Sulpiride appliqué sur le grain neutre comporte de 0,5 à 5 % en poids de polyvinylpyrrolidone.

5. Nouvelles formes galéniques du Sulpiride, selon une des revendications 1 à 4, caractérisées en ce que la couche contenant le Sulpiride contient du talc en faibles proportions.

6. Procédé de préparation des nouvelles formes galéniques du Sulpiride, selon une des revendications 1 à 5, caractérisées en ce qu'on projette en turbine, au moyen d'une solution alcoolique de polyvinylpyrrolidone, environ 70 % en poids de Sulpiride sur 20 % en poids de grains neutres, que l'on sèche et tamise lesdits microgranules, puis qu'on applique une solution alcoolique de polymères méthacryliques comportant environ 4 parties en poids d'Eudragit (R) du type L et environ 1 partie en poids d'Eudragit (R) du type RL, que l'on sèche avec du talc, puis à l'étuve à 37°C, pendant une période suffisante, avant de terminer la couche externe au moyen d'une solution alcoolique d'Eudragit du type RL, de façon à ajuster la cinétique de libération in vitro du Sulpiride, selon lesdites valeurs caractéristiques.

**Office européen des brevets**

## RAPPORT DE RECHERCHE EUROPEENNE

Numéro de la demande

EP 84 40 0661

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 454 804 (PARCOR)<br><br>* Revendications 1,6,10 * | 1,4,5,<br>6 | A 61 K 9/54<br>A 61 K 31/40 |
| X | WO-A-8 201 649 (LARUELLE)<br>* Revendications; page 6, ligne .2 - page 7, ligne 8 * | 1 | |
| Y | EP-A-0 040 590 (A.B. HÄSSLE)<br>* Revendications 1,2,4,5; page 3, ligne 11 - page 4, ligne 2; page 4, lignes 26-31; page 11, tableau: exemple 3 * | 1-3 | |
| X | | 6 | |
| E | EP-A-0 107 557 (LARUELLE)<br>* Revendications; page 2, lignes 25-33; exemple 1 * | 1,4,5 | DOMAINES TECHNIQUES RECHERCHES (Int. Cl.4) |
| D,Y | FR-A-2 453 642 (LABAZ)<br>* Revendications; exemple 1; page 6, ligne 35 - page 7, ligne 29 * | 1-3 | A 61 K 9/00 |

Le présent rapport de recherche a été établi pour toutes les revendications

| Lieu de la recherche | Date d'achèvement de la recherche | Examinateur |
|---|---|---|
| LA HAYE | 21-02-1985 | WILLEKENS G.E.J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82